# FASCICULE DE BREVET EUROPEEN

(11) **EP 2 442 838 B1**
(45) Date de publication et mention de la délivrance du brevet: **08.04.2015**
(21) Numéro de dépôt: 10734250.3
(22) Date de dépôt: 16.06.2010
(51) Int. Cl.: A61L 27/36, A61K 35/56

(54) **PROCÉDÉ DE PRÉPARATION DE NACRE MÉCANOSTRUCTURÉE PAR MÉCANO-SYNTHÈSE, NACRE MÉCANOSTRUCTURÉE AINSI OBTENUE ET SES APPLICATIONS**
VERFAHREN ZUR HERSTELLUNG VON MECHANISCH STRUKTURIERTEM PERLMUTT MITTELS MECHANOSYNTHESE, AUF DIESE WEISE HERGESTELLTES MECHANISCH STRUKTURIERTES PERLMUTT UND ANWENDUNG
METHOD FOR PREPARING MECHANICALLY STRUCTURED MOTHER-OF-PEARL BY MECHANOSYNTHESIS, MECHANICALLY STRUCTURED MOTHER-OF-PEARL PRODUCED THEREBY, AND THE APPLICATIONS THEREOF

(30) Priorité: 17.06.2009 FR 0954066
(43) Date de publication de la demande: 25.04.2012
(73) Titulaire: JD Invest, 94100 Saint-Maur- Des-fosses (FR)
(72) Inventeur: CAMPRASSE, Serge., F72200 La Flèche (FR); CAMPRASSE, Georges., F-72350 Chevillé (FR)
(74) Mandataire: Cabinet Plasseraud
(86) Numéro de dépôt international: PCT/FR2010/051201
(87) Numéro de publication internationale: WO 2010/146308

(56) Documents cités:
- WO-A1-01/84963
- FR-A1- 2 777 190
- FR-A1- 2 827 478
- LEMOS A F ET AL: "Hydroxyapatite nano-powders produced hydrothermally from nacreous material" JOURNAL OF THE EUROPEAN CERAMIC SOCIETY, ELSEVIER SCIENCE PUBLISHERS, BARKING, ESSEX, GB, vol. 26, no. 16, 1 janvier 2006 (2006-01-01), pages 3639-3646, XP024960271 ISSN: 0955-2219 [extrait le 2006-01-01]
- DATABASE WPI Week 200825 Thomson Scientific, London, GB; AN 2008-D29692 XP002565641 & CN 101 062 060 A (HAINAN JINGRUN PEARL BIOTECHNOLOGY LTD) 31 octobre 2007 (2007-10-31)
- GAO ET AL: "Effect of nanometer pearl powder on calcium absorption and utilization in rats" FOOD CHEMISTRY, ELSEVIER LTD, NL, vol. 109, no. 3, 25 décembre 2007 (2007-12-25), pages 493-498, XP022534617 ISSN: 0308-8146
- KEITH J ET AL: "Comparative analysis of macromolecules in mollusc shells" COMPARATIVE BIOCHEMISTRY AND PHYSIOLOGY. B. COMPARATIVEBIOCHEMISTRY, PERGAMON PRESS, LONDON, GB, vol. 105B, no. 3/04, 1 janvier 1993 (1993-01-01), pages 487-496, XP002089924 ISSN: 0305-0491

## Description

### Domaine technique

La présente invention concerne un procédé de préparation de nacre mécanostructurée par mécano-synthèse. Elle concerne également les applications de cette nacre, notamment en tant que revêtement disposé sur des implants, des prothèses métalliques ou pièces de comblement osseux.

### Etat de la technique

La nacre est un complexe organominéral sécrété tout au long de leur vie par certains mollusques. Elle est principalement composée de carbonate de calcium cristallisé sous forme d'aragonite très pure disposé en couches superposées, séparées par des couches de matière organique. L'ensemble de ces parties minérales et organiques illustre la complexité de sa composition.

Afin de mieux comprendre les raisons qui rendent la nacre si particulière, il a été identifié, dans le brevet WO9952940, certaines molécules biologiques des coquilles externes et internes de mollusques marins bivalves. Pour cela, de la poudre de coquille externe et interne de Tridacnae Gigas et de Pinctada Maxima a été préparée, en découpant des fragments de quelques cm² de coquille puis en les broyant pendant deux phases de 3 minutes dans un broyeur planétaire. La poudre obtenue présentait une granulométrie entre 300 et 500 microns. Les principes actifs ou biopolymères marins contenus dans la nacre ont alors été extraits par hydrolyse à froid, supercentrifugation et ultra-filtration tangentielle. Bien que cette méthode d'extraction ne garantisse pas que tous les composants soient effectivement retenus, ils ont identifié des protéines de type structural constitutives de la matrice extra cellulaire, telles que des acides aminés essentiels, du collagène de type I, II et III (glycoprotéines fibreuses très abondantes dans la matière organique), de l'élastine, des glycosaminoglycanes et des protéoglycanes. Ils ont également mis en évidence la présence de pro-facteurs et de facteurs de croissance tels que BMP, TGFβ, IGFII, de cytokines, de lipides et d'hexoses (sucres réducteurs en C6 indispensables dans le métabolisme cellulaire), de composés mélaniques et de caroténoïdes ainsi que des éléments minéraux et métalliques libres ou associés à des molécules biologiques spécifiques pour former des métallo-protéines, des métallo-enzymes, des chromo-protéines porphyriniques et non porphyriniques, formant les 2/3 des éléments constitutifs de matrice organique.

De nombreuses études et relevés d'observations cliniques ont, non seulement montré l'excellente biocompatibilité des biopolymères marins, mais encore toutes leurs propriétés pharmacologiques dans les indications où elles ont été utilisées. Ainsi, le pouvoir de régénération cutanée mais aussi de régénération de l'os aussi bien spongieux que compact, in vivo et in vitro, de la nacre a été démontré. C'est sa composition spécifique et sa structure particulière qui confèrent à la nacre compacte le caractère histocompatible et non biodégradable mais aussi des propriétés mécaniques constantes et comparables à celles de la dent naturelle, de l'os humain et des céramiques les plus résistantes. Ainsi, la composition physico-chimique de la nacre en fait le biomatériau le mieux adapté à une implantation endo-osseuse sous forme compacte, tel que décrit dans le brevet FR2647334 en proposant l'utilisation de la nacre dans des pièces osseuses de substitution et radiculo-dentaire mais également sous forme pulvérulente dans d'autres applications telles que la cicatrisation des pertes de substances cutanées, musculaires et dans le comblement de pertes de substances osseuses.

Cependant, la nacre de ces mollusques utilisée sous forme compacte comme implant endo-osseux, plaques et vis d'ostéosynthèses ou pièces de substitution osseuse, de par sa faible porosité uniquement de surface (publications Académie des Sciences, Clinical Material) avec des pores non communicants, ne permet qu'une libération limitée des principes actifs ostéo-inducteurs responsables de l'ostéogénèse.

Il existe donc un besoin d'amélioration ou d'optimisation des propriétés de la nacre, ou encore, un besoin de lui conférer de nouvelles propriétés permettant de nouvelles applications.

La nacre aragonitique de la coquille de bivalves, tels que Pinctada Maxima ou autres Pinctadines et Tridacnae Gigas, possède une microstructure cristalline la rapprochant d'un nano-composite naturel. En effet, le composant élémentaire de la nacre est un cristal biogénique, organominéral et aragonitique qui est associé et lié à d'autres biocristaux par la matière organique résultant de la synthèse glycoprotéique de cellules spécialisées. Ces biocristaux sont entourés et séparés par de la matière organique nanostructurée, les fibrilles qui la composent présentant des tailles allant de 10 à 100 nanomètres selon qu'elles soient intercristallines ou interlamellaires.

Compte tenu des propriétés intrinsèques des biopolymères contenus dans la nacre des mollusques cités précédemment, de la présence de minéraux et surtout de métaux libres ou associés à des molécules protéiques tels que les métallo-protéines, les métallo-enzymes, les métallo-porphyrines, les présents inventeurs ont alors envisagé, pour optimiser les propriétés de la nacre, de la transformer par mécano-synthèse en particules de nacre mécanostructurée.

Ils ont ainsi trouvé qu'il était possible d'obtenir de la nacre mécanostructurée par un procédé de mécanosynthèse.

### Exposé de l'invention

Ainsi, l'invention a pour objet un procédé de préparation de nacre mécanostructurée par mécanosynthèse. Elle a également pour objet l'application de cette nacre mécanostructurée dans les domaines médicaux, vétérinaires ou cosmétiques et notamment dans la conception de prothèses osseuses ou dentaires.

Les attributs les plus importants définissant les caractéristiques des nanoparticules sont leur taille. En effet, les matériaux présentant des tailles de particules de l'ordre du nanomètre ont la particularité d'avoir des propriétés physico-chimiques particulières, propres aux dimensions de l'infiniment petit. Ainsi, les propriétés de la matière peuvent se modifier et/ou s'accentuer lorsque la taille des particules se rapproche du nanomètre, par exemple, leur surface de réaction chimique est plus élevée et la faible taille des nanoparticules leur procure une plus grande facilité à franchir les barrières biologiques. Le cas échéant, les effets pharmacologiques peuvent être augmentés. Elles peuvent donc agir au sein de la cellule en franchissant les membranes plasmiques aussi bien sur le cytosquelette que sur les organites.

C'est ainsi que toutes les propriétés pharmacologiques, biologiques et biochimiques d'une substance contenant des nanoparticules peuvent être majorées.

A ces avantages liés à la taille des particules, s'ajoutent les avantages liés au procédé de mécano-synthèse mis en oeuvre dans l'invention. En effet, sans vouloir être liés par une quelconque théorie, les inventeurs sont d'avis que, en raison notamment de la présence de métallo-protéines et métallo-porphyrines et de métallo-enzymes dans la nacre, une réorganisation des différents éléments constitutifs entre eux est réalisée lors de la mécano-synthèse, réorganisation qui n'est pas obtenue avec un broyage classique.

En particulier, compte tenu de la présence des métaux libres ou associés dans la composition moléculaire du test nacré, c'est-à-dire de la coquille interne du mollusque bivalve, notamment Mn, Cl, Cu, K, Sr, Na, Zn, Br, Ce, Fe, La, Sm et compte tenu de leurs rôles comme coenzymes dans tous les systèmes biologiques, dans les réactions catalytiques d'hydrolyse et de transfert d'électrons dans les métallo-protéines à transfert d'électrons, de transfert et d'activation du dioxygènes, les métallo-enzymes catalytiques, les inventeurs sont d'avis que l'application d'un procédé de chimie bioinorganique du type descendant (Top Down), peut conférer à tous ces éléments des propriétés majorées faisant de l'ensemble un nouveau biocomposite présentant des propriétés de régénération tissulaire et cellulaire améliorées.

Les présents inventeurs ont découvert que le maintien de la nacre à une température inférieure à 40°C, de préférence inférieure à 20°C, et plus préférentiellement encore inférieure ou égale à 0°C pendant le procédé de préparation par mécano-synthèse permettait d'obtenir de la nacre mécanostructurée montrant des propriétés améliorées et conservant l'ensemble de ses constituants protéiniques.

### Description de l'invention

Ainsi, la présente invention a pour objet un procédé de préparation de nacre mécanostructurée par mécano-synthèse de poudre micrométrique de nacre provenant du test nacré, caractérisé en ce que la température de la nacre est maintenue inférieure à 40°C, de préférence inférieure à 20°C, et plus préférentiellement encore inférieure ou égale à 0°C. Dans la présente demande et dans ce qui suit, le préfixe "nano" sera utilisé pour décrire des particules dont le diamètre moyen en volume est inférieur à 500 nanomètres, de préférence inférieur à 250nm, et de plus préférentiellement inférieur ou égal à 100nm. Par conséquent, les termes de "nanoparticules" et de "particules nanonisées" seront utilisés pour décrire des particules dont le diamètre moyen en volume est inférieur à 500 nanomètres, de préférence inférieur à 250nm, et de plus préférentiellement inférieur ou égal à 100nm.On entend par "poudre micrométrique", une poudre présentant des particules dont le diamètre moyen en volume est compris entre 1 et 500 µm, de préférence entre 1 et 100 µm et plus préférentiellement entre 1 et 20 µm.

De plus, les termes "particules de nacre à traiter" ou "poudre de nacre à traiter" doivent être considérés comme se rapportant aux particules de nacre ou à la poudre de nacre telles qu'avant l'application du procédé de mécano synthèse. De même, les termes "particules mécanostructurées de nacre" ou "nacre mécanostructurée" ou "nacre mécanoinduite" feront référence à la poudre de nacre telle qu'ayant subi le procédé selon l'invention.

Le diamètre moyen équivalent en volume des poudres de l'invention est déterminé par diffraction laser à l'aide d'un granulomètre laser. A partir de la répartition granulométrique mesurée sur une large gamme, le diamètre moyen équivalent en volume ou D(4;3) est calculé selon la formule suivante : D(4;3) = ∑ (d⁴) / ∑ (d³).

La mécano-synthèse (ou synthèse par voie mécanique) est une méthode mécanique qui consiste à broyer des poudres micrométriques, permettant, sous l'effet d'une succession de chocs mécaniques sur les particules à l'intérieur d'un conteneur, d'obtenir des matériaux modifiés sous forme de poudre de taille nanométrique.

Le diamètre moyen en volume de la poudre de nacre issue du procédé de mécanosynthèse est inférieur à 500nm, de préférence inférieur à 250nm et plus préférentiellement inférieur ou égal à 100nm.

On sait qu'en chimie bioinorganique, le broyage et le co-broyage réalisés par le déplacement de billes de broyage dans un espace clos soumis lui-même à des forces d'accélération importantes et appliqués à des matériaux métalliques pulvérulents de différentes natures conduisent à la production de nanoparticules et à la synthèse de nouveaux composés aux propriétés nouvelles par la mise en oeuvre de réactions chimiques induites par la haute énergie mécanique provoquée par le type de broyage mis en oeuvre.

Les éléments métalliques présents dans la matière organique de Pinctada Maxima ou autres Pinctadines et de Tridacnae Gigas tels que : Sm, La, Zn, Br, Ce, Fe, Mn, Cu, K, Sr, Na et Ca sous forme libre ou associés à des protéines enzymatiques et non enzymatiques, à des porphyrines, font de l'aragonite de ces mollusques un système biologique particulier et unique propre à une application en chimie bioinorganique ou biomimétique, discipline étudiant la dynamique des cations métalliques dans les systèmes biologiques.

On voit donc que tous les ions métalliques contenus dans la matière organique de l'aragonite de Pinctada Maxima et autres Pinctadines et de Tridacnae Gigas ont un rôle déterminant dans le métabolisme cellulaire à tous les niveaux : canaux ioniques, concentration des cations métalliques cellulaires, réactions d'hydrolyse, régénération du cytosquelette, transfert d'électrons, transport et activation du dioxygène, inhibition du stress oxydatif jouant un rôle capital dans l'homéostasie cellulaire et tissulaire.

L'utilisation d'un broyeur planétaire à paramètres découplés est particulièrement bien adaptée pour la synthèse par voies mécaniques des nanomatériaux. Le broyeur planétaire est constitué d'un plateau central sur lequel sont fixés des satellites, l'accélération centrifuge étant modulable en fonction des conditions de rotation relative du plateau et des satellites.

La nacre étant un matériau très dur, il est important que durant le broyage aucune particule issue du broyeur ou des billes ne vienne contaminer la poudre de nacre ainsi obtenue. C'est pourquoi, le ou les bols de broyage ainsi que les billes de broyage utilisés selon l'invention doivent être constitués d'un matériau plus dur que la nacre, biocompatible et non polluant. On peut citer comme matériau, par exemple l'oxyde de zirconium ou un alliage zirconium-yttrium, ces matériaux ne relargueront pas d'éléments chimiques lors des chocs violents et répétés avec la nacre dans le broyeur.

Dans le procédé de l'invention, on utilisera préférentiellement une poudre de nacre à traiter présentant un diamètre moyen en volume compris entre 1 et 20 microns afin de limiter la durée et le nombre des séquences de broyage.

Selon le procédé de l'invention, c'est le test nacré (c'est-à-dire la coquille interne du mollusque bivalve) de la coquille qui est concassé puis broyé. L'utilisation d'un tel procédé permet de conserver et de traiter la totalité de la matière organique liée aux cristaux de carbonate de calcium biogéniques. Sans vouloir être liés par aucune théorie, les présents inventeurs sont d'avis que le procédé de mécano-synthèse dans le broyeur planétaire permet le broyage de tous les éléments organiques et métalliques du biomatériau, et provoque l'arrachement, l'écrasement, la cohésion, et la décohésion, la déformation plastique et élastique des différentes nano-particules en induisant la synthèse de nouvelles molécules où les métalloprotéines, métallo-enzymes et métallo-porphyrines sont agrégées soit entre elles par leurs groupements prosthétiques, soit avec d'autres protéines ou encore avec d'autres ions métalliques libres, créant de nouvelles séquences d'acides aminés donnant naissance à des oligo-peptides, des polypeptides, des peptides ou des protéines dotés de nouvelles propriétés physico-chimiques et biologiques.

La coquille interne est utilisée telle quelle, elle est concassée et broyée, et n'a subi aucun traitement préalable, à l'exception d'une décontamination notamment à l'eau de javel (hypochlorite), ou autre décontaminant, par exemple un ammonium quaternaire, le calbénium, puis d'un rinçage à l'eau.

La nacre mise en oeuvre dans le procédé de l'invention provient du test nacré de la coquille de bivalve choisi dans le groupe comprenant la Pinctada Maxima, la Pinctada Margaritifera ou autres Pinctadines, le Tridacnae Gigas, et leurs mélanges.

Selon un mode de réalisation particulier, l'invention concerne un procédé de préparation de nacre mécanostructurée par mécano-synthèse de poudre micrométrique de nacre comprenant les étapes successives suivantes :
a) la poudre de nacre à traiter est placée dans un bol de broyage d'un broyeur planétaire, puis
b) Nᵢ billes de broyage (avec i est un nombre entier compris entre 1 et 20, de préférence entre 3 et 15 et plus préférentiellement entre 5 et 14 ; Nᵢ est un nombre entier compris entre 2 et 150, de préférence entre 10 et 100 et plus préférentiellement entre 20 et 85) de diamètre Dᵢ sont placées dans le bol de broyage,
c) le broyeur planétaire est mis en route, à une vitesse de rotation V comprise entre 800 et 1400 tr/min, préférentiellement à 1100 tr/min, avec une accélération de 90 à 110 G, de préférence de 90 à 100 G et plus préférentiellement de 95 G,
d) le broyeur planétaire est arrêté et les billes de broyage de diamètre Dᵢ sont retirées,
les étapes b, c et d sont répétées avec Nᵢ₊₁ (avec Nᵢ₊₁ > Nᵢ) billes de broyage de diamètre Dᵢ₊₁(avec Dᵢ₊₁ < Dᵢ), jusqu'à l'obtention de particules de nacre de la taille souhaitée ;
lorsque la taille souhaitée des particules est obtenue, à l'issue de l'étape d), la nacre mécanostructurée est récupérée.

Ainsi, le choix du nombre de séquences de broyage dépendra de la taille de particules souhaitée. Le diamètre des billes est compris entre 1 et 30 mm; de préférence entre 1 et 10 mm en plus préférentiellement entre 1 et 5 mm. Le nombre et le diamètre des billes dépendront de la taille du bol de broyage. Par exemple, avec un bol de broyage ayant un volume de 500 ml, on peut envisager l'utilisation de 25 billes de 20 mm de diamètre, ou 50 billes de 10 mm de diamètre ou encore 80 billes de 5 mm de diamètre.

Selon un mode de réalisation particulièrement préféré, le nombre de billes et leur diamètre sont tels que l'on ait un rapport 2/5 de poids de particules de nacre à traiter pour 3/5 de poids de billes. Cette proportion est tout à fait adaptée à un broyage dans un bol de broyage dont le volume utile est de 500mL.

Par volume utile, on entend la contenance du bol de broyage vide et fermé.

De plus, plus les billes présentent un faible diamètre, plus les chocs sont multiples et plus la mecanosynthèse est rapide.

Selon un mode de réalisation avantageux, des billes de 2mm de diamètre sont utilisées.

Le maintien de la nacre à une température inférieure à 40°C, de préférence inférieure à 20°C, et plus préférentiellement encore inférieure ou égale à 0°C, pendant tout le procédé de mécano-synthèse est nécessaire à la non altération de la structure tertiaire des éléments organiques et notamment des protéines composant la nacre.

Ainsi, selon un mode particulier de l'invention, le bol de broyage, la poudre de nacre à traiter et/ou les billes de broyage sont refroidis avant utilisation à une température comprise entre -30°C et 5°C, de préférence entre -20°C et -15°C, et éventuellement avant chaque répétition des étapes b), c) et d).

Le bol de broyage, la poudre de nacre à traiter et/ou les billes de broyage peuvent ainsi être placés dans un congélateur à une température comprise entre -30°C et 5°C, de préférence entre -20°C et -15°C pendant une durée allant de 1 minute à 48 heures. Plus préférentiellement, l'ensemble comprenant le bol de broyage, la poudre de nacre à traiter et/ou les billes de broyage est placé dans un congélateur à une température de -18°C pendant 24 heures. Par ailleurs, ce refroidissement préalable permet d'augmenter la teneur en eau de la nacre et ainsi faciliter le broyage et la mécanosynthèse. La teneur en eau qui initialement est de 0,5% d'humidité relative pour la nacre micronisée mise en oeuvre comme produit de départ, peut augmenter jusqu'à 5% d'humidité relative lors des séquences de refroidissement.

De même, le broyage de l'étape c) pourra être entrecoupé de séquences de refroidissement des billes de broyage, du bol de broyage et/ou de la poudre de nacre à traiter afin de limiter l'augmentation de température subie par la poudre de nacre pendant le broyage.

Ainsi, selon un mode de réalisation particulier de l'invention, le procédé de mécano-synthèse de poudre de nacre est caractérisé en ce que les séquences de broyage c) sont effectuées sous atmosphère refroidie ou entrecoupées de séquences de refroidissement.

Il est également possible de doter le broyeur planétaire d'un dispositif de ventilation, destiné à contrebalancer la réaction exothermique résultant de la haute énergie générée par la vitesse de rotation de l'ensemble et par les chocs des billes à grande vitesse, ou bien de conduire le procédé ou au moins l'étape c) sous azote liquide.

La nacre mécanostructurée obtenue à l'issue du procédé de l'invention peut être stérilisée, par exemple par rayonnement gamma à moins de 25 kGy ou à l'oxyde d'éthylène pendant 24h suivi de 24h d'aération.

Selon un autre mode de réalisation particulier de l'invention, le procédé de mécano-synthèse de particules de nacre est caractérisé en ce que :
- la poudre de nacre à traiter est déposée dans un bol de broyage, des billes de broyage sont déposées dans le bol de broyage et l'ensemble comprenant la poudre de nacre à traiter, les billes de broyage et le bol de broyage est refroidi à une température comprise entre -30°C et 5°C, de préférence entre -20°C et -15°C,
- la poudre de nacre à traiter réfrigérée subit ensuite :
   a) des séquences de broyage avec des billes de broyage de 10 mm de diamètre jusqu'à l'obtention de particules de nacre ayant un diamètre moyen en volume compris entre 5 et 15 microns, puis
   b) des séquences de broyage avec des billes de broyage de 5 mm de diamètre, jusqu'à l'obtention de particules de nacre ayant un diamètre moyen en volume compris entre 800 nanomètres et 2 microns, puis
   c) des séquences de broyage avec des billes de broyage de 2 mm de diamètre, jusqu'à l'obtention de particules de nacre ayant un diamètre moyen en volume compris entre 0,01 et 500 nanomètres, de préférence entre 0,01 et 250 nm et plus préférentiellement entre 0,01 et 100 nm,
   la nacre mécanostructurée est alors décollée de la paroi du bol et des billes, tamisée et récupérée.

Le broyage est réalisé par séquences de façon à limiter l'échauffement de la nacre.

Selon un autre mode de réalisation, la présente invention a pour objet un procédé de mécano-synthèse de particules de nacre caractérisé en ce que :
- la poudre de nacre à traiter est déposée dans un bol de broyage, des billes de broyage sont déposées dans le bol de broyage et l'ensemble comprenant la poudre de nacre à traiter, les billes de broyage et le bol de broyage est refroidi à une température comprise entre -30°C et 5°C, de préférence entre -20°C et -15°C,- la poudre de nacre réfrigérée subit ensuite :
   a) 5 à 15, de préférence 7 à 13 séquences, et plus préférentiellement encore 8 à 11 séquences de broyage, chaque séquence ayant une durée de 1 à 10 minutes, de préférence de 2 à 8 minutes et plus préférentiellement de 6 minutes, effectuées avec des billes de broyage de 10 mm de diamètre, puis
   b) 5 à 15 séquences de broyage, préférentiellement 10 séquences, de 1 à 10 minutes, préférentiellement 6 minutes, effectuées avec des billes de broyage de 5 mm de diamètre, puis
   c) 5 à 15 séquences de broyage, préférentiellement 10 séquences, de 1 à 10 minutes, préférentiellement 6 minutes, effectuées avec des billes de broyage de 2 mm de diamètre,
   la nacre mécanostructurée est alors décollée de la paroi du bol et des billes, tamisée et récupérée.

Selon un mode particulièrement préféré, le procédé de mécanosynthèse de particules de nacre est caractérisé en ce que :
- la poudre de nacre à traiter est déposée dans un bol de broyage, des billes de broyage de 2mm de diamètre sont déposées dans le bol de broyage et l'ensemble comprenant la poudre de nacre à traiter, les billes de broyage et le bol de broyage est refroidi à une température comprise entre -30°C et 5°C, de préférence entre -20°C et-15°C ;
- la poudre de nacre réfrigérée subit ensuite 20 séquences de broyage de 5 minutes avec les billes de broyage jusqu'à l'obtention de particules de nacre ayant une granulométrie moyenne en volume inférieure à 500nm, de préférence inférieure à 250nm et plus préférentiellement inférieure ou égale à 100nm,
- après chaque séquence, la poudre est décollée de la paroi des bols et tamisée puis la poudre, les bols et les billes sont congelés pendant 24h à une température comprise entre -30°C et 5°C, de préférence entre -20°C et -15°C ;
- après la dernière séquence, la nacre mécanostructurée est alors décollée, tamisée et récupérée.

L'invention porte également sur de la nacre mécanostructurée pouvant être obtenue par le procédé décrit ci-dessus qui présente un diamètre moyen en volume compris entre 0,01 et 500 nanomètres, de préférence entre 0,01 et 250 nm et plus préférentiellement entre 0,01 et 100 nm.

La nacre mécanostructurée issue du procédé de mécanosynthèse est caractérisée par diffraction des rayons X, par spectroscopie Raman et par granulométrie laser afin d'accéder à des informations qualitatives et quantitatives. Ces dernières montrent de façon aléatoire, la présence de carbonate de calcium sous forme cristalline, ainsi qu'une très faible quantité sous forme amorphe qui s'explique par une augmentation de la pression à l'intérieur du bol (environ inférieure à 10GPa) pendant un court instant.

Selon un mode de réalisation de l'invention, la poudre de nacre à traiter est broyée conjointement avec au moins un matériau autre que la nacre.

Cet autre matériau que la nacre est choisi dans le groupe comprenant la poudre de chitosan desacétylé à plus de 90%, la chitine, les algues, les biopolymères insolubles et solubles extraits du test nacré et de la coquille externe des bivalves précités, le sulfate de cuivre (CuSO₄, 5H₂O), l'oxyde de zinc, l'or ou l'argent, et leurs mélanges. Comme sulfate de cuivre, on peut utiliser le sulfate de cuivre pentahydraté, le sulfate de cuivre cristallisé ou sublimé. On peut citer comme exemple préférentiel, le broyage simultané de poudre de nacre présentant un diamètre moyen en volume compris entre 1 et 20 microns et de poudre de chitosan desacétylé à plus de 90% d'un diamètre moyen en volume d'environ 150 microns et présentant une densité de 0,6 g/cm³. Le traitement simultané de ces deux poudres par le procédé de mécanosynthèse de l'invention donne naissance à un nouveau biomatériau mécanostructuré dans lequel la forme tridimensionnelle des particules de chitosan permet une cohésion étroite avec les biocristaux d'aragonite mécano-induits.

La nacre mécanostructurée et/ou le biomatériau mécanostructuré selon l'invention peuvent être utilisés dans de nombreuses applications, telles que les applications médicales, vétérinaires ou cosmétiques. Ainsi, selon un mode de réalisation particulier de l'invention, le biomatériau mécanostructuré issu du broyage simultané de poudre de nacre et de poudre de chitosan desacétylé à plus de 90% obtenu par le procédé selon l'invention entre dans la composition de préparations cosmétiques, notamment pour le traitement anti-âge, de poudre, de fond de teint traitant, de rouge à lèvres, de soins des ongles, de déodorants et de soins capillaires.

La nacre mécanostructurée et/ou le biomatériau mécanostructuré selon l'invention peuvent également être utilisés dans d'autres domaines tels que les domaines médicaux, pharmaceutiques ou vétérinaires, notamment dans la formulation de collyre, de gel et de crème ophtalmiques, éventuellement en association avec des substances médicamenteuses tels qu'antibiotiques, anti-inflammatoires, vasodilatateurs.

Ainsi, préférentiellement, la nacre mécanostructurée et/ou le biomatériau mécanostructuré selon l'invention seront utilisés en application dentaire, par exemple en comblement osseux, implant, pièce de substitution osseuse ou encore, pour traiter les parodontopathies ainsi que comme additifs aux résines polymérisables pour la restauration esthétique coronaire. Ainsi, par exemple, la nacre mécanostructurée et/ou le biomatériau mécanostructuré obtenus par le procédé selon l'invention peuvent entrer dans la composition de pâte à canaux pour les traitements endodontiques, de ciment de coiffage pulpaire et de fond de cavité. Elle (ou il) peut alors être associé(e) à des composants tels que l'oxyde de zinc, l'hydroxyde de calcium, l'eugénol ou toute autre huile essentielle.

La présente invention a également pour objet un implant, comprenant un noyau en matériau de structure, éventuellement en nacre ou un élément prothétique en métal ou autre matériau, à la surface duquel est déposé(e) en revêtement par pulvérisation, projection, enrobage, électrolyse ou trempage, de la nacre mécanostructurée et/ou du biomatériau mécanostructuré selon l'invention.

Cet implant, ou élément prothétique, est de taille et de diamètre variables et est caractérisé en ce qu'il est recouvert sur toute sa surface par de la nacre mécanostructurée et/ou un biomatériau mécanostructuré, tel que décrit dans l'invention. La réalisation d'un revêtement à partir de nacre mécanostructurée et/ou de biomatériau mécanostructuré permet ainsi de lui conférer de nouvelles fonctionnalités. En effet, compte tenu de l'ensemble des propriétés des nanoparticules mécano-induites, il devient possible par traitement de surface d'un matériau, par projection ou enrobage par des particules mécanostructurées :
- de modifier sa rugosité de surface pour un meilleur ancrage,
- d'augmenter son interface avec le site récepteur sans en modifier ses formes et dimensions, afin de rendre immédiatement biodisponibles toutes les molécules dotées de propriétés pharmacologiques intervenant dans les processus de régénération et de cicatrisation et enfin,
- d'accélérer l'interactivité biomatériau-cellules, afin de faciliter l'orientation fonctionnelle des cellules et tissus du site receveur ainsi que d'augmenter sa biocompatibilité et sa fonctionnalité.

Dans la suite du texte, les termes « implant » ou « élément prothétique » seront utilisés indifféremment.

La présente invention concerne donc également un implant préférentiellement en nacre, caractérisé en ce qu'il est recouvert partiellement ou sur toute sa surface de nacre mécanostructurée et/ou de biomatériau mécanostructuré préparée selon le procédé de l'invention, par un procédé permettant le dépôt des nanoparticules mécano-induites, tel que la pulvérisation, la projection, l'enrobage, l'électrolyse ou le trempage.

Ainsi, selon un mode préféré de l'invention, la nacre mécanostructurée et/ou le biomatériau mécanostructuré sont appliqués en revêtement par pulvérisation, projection, enrobage, électrolyse ou trempage.

Selon un aspect particulier de l'invention, la nacre mécanostructurée et/ou le biomatériau mécanostructuré préparé(e) selon le procédé de l'invention est utilisé(e) en revêtement d'implant osseux, dentaires, de pièces de substitution osseuse.

L'invention sera mieux comprise si l'on se réfère aux exemples et dessins annexés qui sont non limitatifs, sur lesquels les figures 1 à 3 représentent deux modes de réalisation préférés de l'invention :
- la figure 1 représente une vue schématique d'un implant ;
- la figure 2 représente une vue schématique d'une pièce de substitution osseuse ;
- la figure 3 représente la vis de fixation de la figure 2.

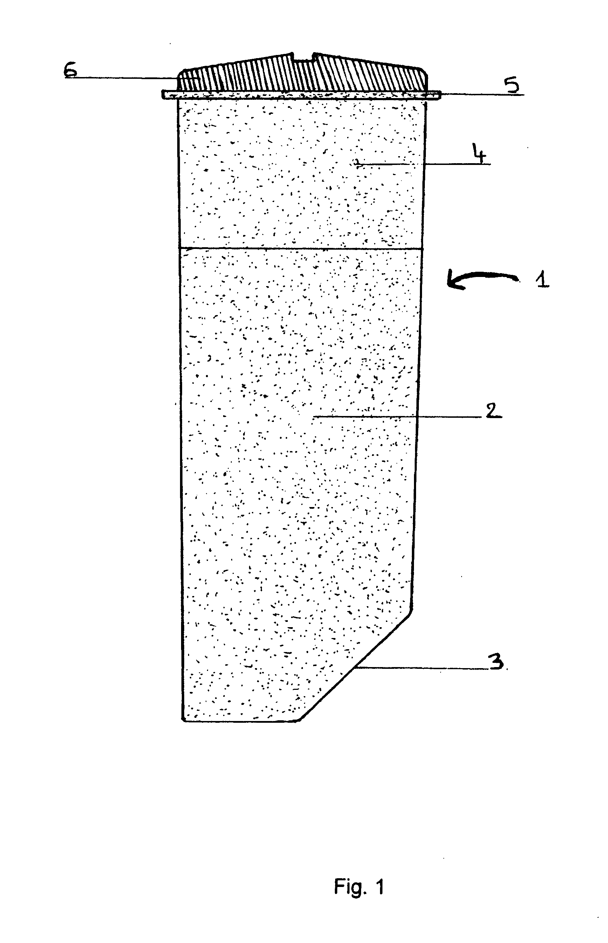

L'implant (1) de la figure 1 comporte une partie de forme générale cylindrique (2), par exemple, d'environ 10 mm, dont l'extrémité inférieure est taillée en biseau (3). Une suprastructure en polyoxyméthylène commercialisé sous la marque Delrin^{®} (4) est vissée sur l'extrémité supérieure de l'implant, et comporte sur son pourtour supérieur un anneau en feutre de polyester commercialisé sous la marque Dacron^{®} (5) par exemple d'1 mm de largeur environ et de 2 mm de hauteur. La suprastructure (4) comporte en son centre un orifice fileté muni d'une vis obturatrice (6). L'ensemble implant-vis obturatrice est recouvert totalement par projection pulvérisation, électrolyse, enrobage, de nacre mécanostructurée préparée selon le procédé de l'invention. Cependant, seule une partie de l'implant présenté sur la figure 1, notamment la partie (2) et/ou la partie (4), peut être recouverte. L'enrobage peut également être fait avec le biomatériau mécanostructuré de l'invention.

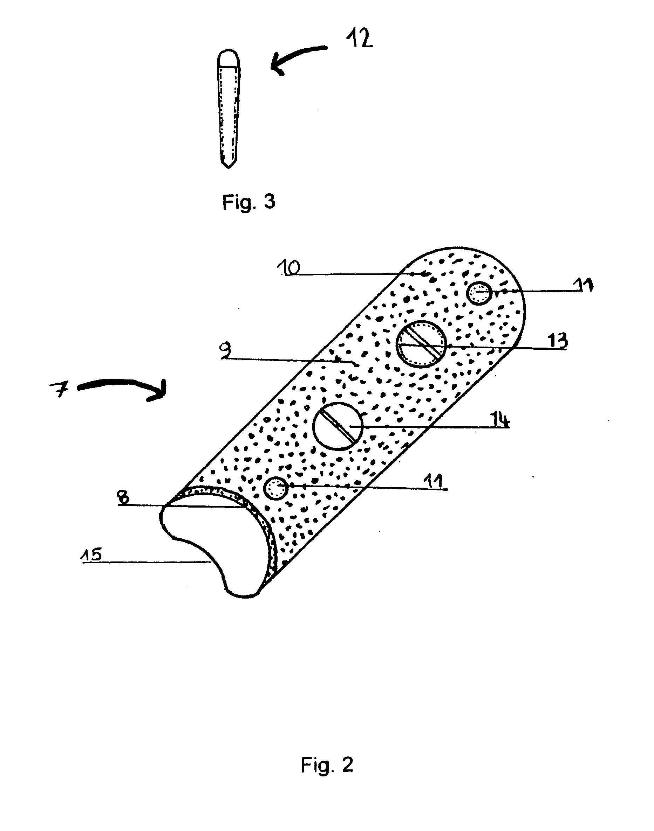

Selon un autre mode de réalisation, le produit selon l'invention représenté sur les figures 2 et 3 est une pièce de substitution osseuse (7) destinée à combler toute perte de substance osseuse au niveau maxillaire. Le produit présente la forme générale d'un parallélépipède de dimensions variables dont la face supérieure convexe (8) est recouverte d'une membrane en feutre de polyester commercialisé sous la marque Dacron^{®} (9) s'arrêtant au niveau des bords arrondis du parallélépipède (10). Ses deux extrémités sont percées de 2 orifices filetés (11), par exemple d'environ 2 mm de diamètre, destinés à permettre sa fixation sur l'os résiduel à l'aide de deux vis (12) (figure 3). Ces vis sont de même nature que le biomatériau de l'implant. Suivant sa longueur, la pièce de substitution osseuse est percée d'un ou plusieurs orifices filetés (13), par exemple de 4 mm de diamètre, destinés à recevoir une suprastructure en matériau synthétique biocompatible, par exemple en polyoxyméthylène commercialisé sous la marque Delrin^{®} et munis de vis obturatrices (14). La suprastructure est destinée à supporter l'élément de restauration prothétique. Sa face inférieure concave (15) est destinée à épouser la surface de l'os résiduel. L'ensemble pièce de substitution osseuse / vis de fixation est recouvert totalement par projection pulvérisation, électrolyse, enrobage ou de nacre mécanostructurée selon l'invention. Cependant, seule une partie de l'implant présenté sur la figure 2 peut être recouverte. Bien entendu, le traitement de surface peut également être fait avec le biomatériau mécanostructuré selon l'invention.

Ainsi, la présente invention a également pour objet une pièce de comblement osseux comprenant un noyau en matériau de structure, éventuellement en nacre, à la surface duquel est déposée en revêtement, par pulvérisation, projection, enrobage, électrolyse ou trempage, de la nacre mécanostructurée et/ou du biomatériau mécanostructuré selon l'invention.

Le produit selon l'invention peut également se présenter sous la forme de plaque et de vis d'ostéosynthèse en matériau de structure, préférentiellement en nacre sous forme compacte taillée dans l'épaisseur de la coquille, de dimensions variables également recouvertes de nacre mécanostructurée ou d'un biomatériau mécanostructuré. Les vis et plaques taillées dans la nacre sous forme compacte présentent des caractéristiques physiques telles que la densité, la résilience, la dureté Vickers, la résistance à la compression, le module d'élasticité, sont proches de celle de l'os. Par conséquent, ils ne nécessitent pas de dépose après constitution et remaniement du cal et peuvent donc être maintenus en place de manière définitive, évitant ainsi tout nouvel acte chirurgical. Le produit selon l'invention peut également être compacté sous haute pression dans des moules de forme et dimension variables pour réaliser des pièces osseuses de substitution destinées à remplacer des épiphyses, des diaphyses et des portions des os longs ou autres parties du squelette.

Il est à noter que les implants réalisés selon l'invention peuvent être utilisés en chirurgie orthopédique, maxillo-faciale et odontostomatologique.

L'invention est utilisable chez les mammifères, en particulier chez l'être humain.

### EXEMPLES

Les exemples qui suivent illustrent la présente invention.

### Exemple 1

On prépare de la poudre de nacre micronisée à traiter selon le procédé suivant :
- le test nacré de Pinctada Maxima est lavé et décontaminé à l'eau de javel à 1% puis est traité par un concasseur et réduit en fragments de 10 mm à 1 cm,
- le produit de concassage est ensuite prélevé puis placé dans un bol de broyage en oxyde de zirconium d'un broyeur planétaire,
- 15 billes de broyage en oxyde de zirconium de 30 mm de diamètre chacune sont ensuite placées dans le bol de broyage,
- le broyeur planétaire est ensuite mis en route, à une vitesse de rotation de 400 tr/min pendant 5 minutes, le dispositif tournant alternativement dans le sens des aiguilles d'une montre et inversement,
- le produit de concassage ayant subi le broyage est ensuite tamisé dans une tamiseuse de 200 mm de diamètre comportant 5 tamis de tailles différentes : 250 microns, 150 microns, 100 microns, 50 microns, 20 microns puis un fond de collecte.

La poudre de nacre récoltée dans le fond de collecte présente un diamètre moyen en volume inférieur à 20 microns.

### Exemple 2

On prépare de la nacre mécanostructurée selon le procédé suivant :
a) la poudre de nacre à traiter obtenue à l'exemple 1 est placée dans un bol de broyage d'un broyeur planétaire,
b) 25 billes de oxyde de zirconium de 10 mm de diamètre sont ajoutées dans le bol,
c) l'ensemble comprenant la poudre de nacre à traiter, le bol de broyage, ainsi que les billes d'oxyde de zirconium est placé dans un congélateur à une température de -18°C pendant 24 heures,
d) le broyeur planétaire est mis en route, à une vitesse de rotation de 1100 tr/min pour une accélération de 95 G, pendant 10 séquences de 6 minutes chacune, séparées toutes les 2 séquences par 2 heures de congélation à -18°C,
e) le broyeur planétaire est arrêté et les billes d'oxyde de zirconium de 10 mm de diamètre sont retirées.

Les étapes b), c), d) et e) sont répétées avec 50 billes d'oxyde de zirconium de 5 mm de diamètre, puis avec 80 billes d'oxyde de zirconium de 2 mm de diamètre. La nacre mécanostructurée est alors décollée de la paroi du bol, tamisée et récupérée. Elle présente un diamètre moyen en volume inférieur à 150 nm. Elle est ensuite stérilisée par rayonnement gamma à 25 kGy.

### Exemple 3:

On prépare de la nacre mécanostructurée selon le procédé suivant :
a) la poudre de nacre aragonitique micronisée d'une masse de 200 grammes obtenue à l'exemple 1 est placée dans un bol de broyage d'une contenance de 500 mL,
b) des billes en oxyde de zirconium d'un diamètre de 2mm et d'un poids de 300 grammes sont ajoutées,
c) l'ensemble comprenant la poudre de nacre micronisée, le bol ainsi que les billes en oxyde de zirconium est placé dans un congélateur à une température comprise entre-15° C et -20° C pendant 24 heures,
d) les bols mis en place, le broyeur planétaire est mis en route à une vitesse de rotation de 1100 tours / minutes pour une accélération de 95 G, pendant 20 séquences de 5 minutes chacune séparée toutes les deux séquences par deux heures de congélation à une température comprise entre -15° C et -20° C,
e) à la fin de chaque séquence, on procède au décollage de la poudre de la paroi des bols, à la dernière séquence, la poudre mécanostructurée est décollée, tamisée afin de récupérer les billes

La nacre mécanostructurée est récupérée, conditionnée et stérilisée par rayonnement ionisant ou vapeur d'oxyde d'éthylène pendant 24h suivi de 24h d'aération.

### Exemple 4

On prépare un biomatériau mécanostructuré issu d'un co-broyat de particules de nacre et de chitosan, selon le procédé suivant :
a) 200 g de la poudre de nacre à traiter obtenue à l'exemple 1 sont placés dans un bol de broyage d'une contenance de 500mL d'un broyeur planétaire,
b) la poudre de chitosan désacétylé à plus de 90 %, présentant un diamètre moyen en volume d'environ 150 µm et de masse volumique de 0,6 g/cm³ est ajoutée dans le bol,
c) 300 grammes de billes en oxyde de zirconium de 2 mm de diamètre sont ajoutés dans le bol,
d) l'ensemble comprenant la poudre de nacre à traiter, la poudre de chitosan désacétylé, le bol de broyage, ainsi que les billes d'oxyde de zirconium est placé dans un congélateur à une température de -18°C pendant 24 heures,
e) le broyeur planétaire est mis en route, à une vitesse de rotation de 1100 tr/min pour une accélération de 95 G, pendant 10 séquences de 6 minutes chacune, séparées toutes les 2 séquences par 2 heures de congélation à -18°C,
f) le broyeur planétaire est arrêté et les billes d'oxyde de zirconium de 2 mm de diamètre sont retirées.

Les étapes c), d), e) et f) sont répétées jusqu'à l'obtention d'un co-broyat de nacre et de chitosan désacétylé montrant un diamètre moyen équivalent en volume de l'ordre du nanomètre. Le biomatériau mécanostructuré est alors décollé, tamisé et récupéré. Puis il est stérilisé par rayonnement gamma à 25 kGy.

### Exemple 5

On taille dans la masse de la nacre des plaques et vis que l'on recouvre de nacre mécanostructurée obtenue dans l'exemple 2. La plaque et les deux vis réalisées selon les figures 2 et 3 sont ensuite placées sur le fémur d'une brebis de réforme. L'examen radiologique à un mois montre que la plaque et les vis ont été recouvertes par la corticale de l'os et sont parfaitement intégrées dans le cal remanié.

### Exemple 6

On prépare un substitut osseux, dont la composition pour 100 g est la suivante :
- 96 g de nacre présentant un diamètre moyen en volume compris entre 20 et 350 microns,
- 4 g de nacre mécanostructurée de l'exemple 2.

### Exemple 7

On prépare un ciment de scellement de prothèses de hanche, de genoux ou d'épaule. La composition du ciment de scellement pour 100 g est la suivante :
- 90 g de poudre de nacre présentant un diamètre moyen en volume de 5 à 50 microns,
- 10 g de nacre mécanostructurée de l'exemple 2.

### Exemple 8

On prépare un gel ou une pâte destinée au traitement des parodontopathies en odontostomatologie dont la composition pour 100 g est la suivante :
- 5 g de poudre de nacre présentant un diamètre moyen en volume entre 5 µm et 10 µm,
- 4 g de nacre mécanostructurée de l'exemple 2,
- 0,05 g de chlorhexidine,
- 2 g de gomme xanthane,
- eau déminéralisée qsp 100g.

### Exemple 9

On prépare une pâte pouvant être utilisée pour les traitements endodontiques comme fond de cavité ou en coiffage pulpaire dont la composition pour 100 g est la suivante :
- 15 g de poudre de nacre présentant un diamètre moyen en volume entre 5 µm et 20 µm,
- 5 g de nacre mécanostructurée de l'exemple 2,
- 80g d'oxyde de zinc.

Le mélange ainsi obtenu peut être mixé avec de l'eugénol ou tous autres vecteurs aqueux, huileux ou tout polymère, biocompatible afin de réaliser une pâte fluide à usage extemporané pour obturation des canaux.

### Exemple 10

On prépare un mélange pouvant être utilisé pour l'obturation des défauts de l'émail et de la dentine dont la composition pour 100g est la suivante :
- 80g de résine photopolymérisable de type epoxy,
- 10g d'agent de couplage de type silane,
- 10g de nacre mécanostructurée de l'exemple 2.

### Exemple 11

On prépare une formulation à visée thérapeutique pour les grands brûlés dont la composition pour 100 g est la suivante :
- 7 g de poudre de nacre présentant un diamètre moyen en volume compris entre 1 et 5 microns,
- 3 g de nacre mécanostructurée de l'exemple 2,
- qsp 100 g de Cérat de Gallien, alginate et chitosan.

### Exemple 12

On prépare une crème ou un gel destiné au traitement des plaies cutanées, musculaires ou muqueuses, d'escarres et d'ulcères, dont la composition pour 100 g est la suivante :
- 1,7 g de poudre de nacre présentant un diamètre moyen en volume compris entre 1 et 5 microns,
- 0,3 g de biomatériau mécanostructuré préparé dans l'exemple 3,
- 1 g de complexe d'huiles essentielles,
- qsp 100 g d'eau déminéralisée, vaseline blanche et karité.

Le produit sous forme de crème a été appliqué toutes les 48h chez le cheval dans le cas d'une nécrose du poitrail de 37 cm de haut sur 16 cm de large, ayant provoqué une perte de substance intéressant tout le revêtement cutané ainsi que le tissu cellulaire sous-cutané jusqu'à l'aponévrose des muscles sous-jacents. On a pu noter une réduction de la surface et de la profondeur de la lésion à raison d'1 cm par jour et la guérison au bout de 45 jours sans décoloration du poil.

### Exemple 13

On prépare un soluté injectable par voie intraveineuse pour le traitement des cachexies et de la fonte musculaire, dont la composition pour 20 ml est la suivante :
- 1g de nacre mécanostructurée de l'exemple 2,
- qsp 20 ml de soluté injectable isotonique.

La préparation injectable a été administrée une fois par voie intraveineuse à un cheval cachexique et dénutri traité en milieu vétérinaire. Après 15 jours, la prise de poids de l'animal était de l'ordre de 25 kg.

### Exemple 14

On prépare un matériau de comblement pour les pertes de substances du sabot chez les ongulés telles que seimes, fourmillières et autres pathologies invalidantes du sabot. La composition pour 100 g du matériau de comblement est la suivante :
- 4 g de poudre de nacre présentant un diamètre moyen en volume compris entre 10 et 20 microns,
- 1 g de nacre mécanostructurée de l'exemple 2,
- 4 g de sciure de hêtre,
- gel de silicone neutre monophasé qsp pour 100g.

### Exemple 15

On prépare un implant selon l'invention de la manière suivante :
- l'implant, tel que décrit figure 1, est recouvert de nacre mécanostructurée préparée dans l'exemple 2 ;
- après anesthésie locale et/ou loco régionale ou générale, l'os maxillaire est mis à nu après incision ou à l'aide d'un bistouri circulaire, à l'aplomb du site à implanter ;
- l'os est foré à l'aide d'instruments calibrés de manière à aménager un puits osseux aux dimensions de l'implant qui est inséré jusqu'à la limite du feutre en Dacron^{®} de la suprastructure décrite figure 1, surplombant la corticale ;
- après suture de la fibromuqueuse gingivale, l'implant est laissé enfoui sans mise en charge.

L'examen clinique et radiologique post opératoire à 2 semaines montre une parfaite cicatrisation gingivale ainsi qu'un comblement rapide de l'espace péri-implantaire, par colonisation par les ostéoblastes des pores aveugles de la surface de l'implant.

Une biopsie au niveau de l'anneau de feutre de Dacron^{®} montre sur une coupe histologique, une colonisation des mailles du feutre de Dacron par les fibroblastes, sans présence de cellules inflammatoires, réalisant une véritable sertissure gingivale avec une gencive attachée.

### Exemple 16

On prépare une pièce de substitution osseuse selon l'invention de la manière suivante :
- la pièce de substitution osseuse, telle que décrite sur les figures 2 et 3, est recouverte de nacre mécanostructurée préparée dans l'exemple 2 ;
- après radios et scanners de la zone à opérer, puis anesthésie locale et/ou loco régionale, ou générale on pratique une incision transversale sur la crête maxillaire et un décollement mucopériosté de manière à permettre la pose d'un expanseur en silicone de formes et dimensions semblables à la pièce de substitution. Le remplissage progressif de l'expanseur par du sérum physiologique pendant 3 semaines environ provoque une expansion de la fibromuqueuse gingivale ;
- après dépose de l'expanseur, la pièce de substitution est insérée dans le tunnel ainsi obtenu et fixée à l'aide des vis de fixation, sa face supérieure recouverte par la fibromuqueuse, la face inférieure reposant sur l'os maxillaire dont on aura cruanté la surface ;
- la pièce de substitution est fixée sur la fibromuqueuse par des sutures transversales.

Des examens radiologiques à 4 semaines montrent une parfaite intégration de la pièce et sa fixation par la fibromuqueuse.

## Revendications

1. Procédé de préparation de nacre mécanostructurée par mécano-synthèse de poudre micrométrique de nacre, **caractérisé en ce que** la température de la nacre est maintenue inférieure à 40°C, de préférence inférieure à 20°C, et plus préférentiellement encore inférieure ou égale à 0°C, et **en ce que** les particules de nacre à traiter proviennent du test nacré, c'est-à-dire la coquille interne, de bivalve.

2. Procédé de préparation de nacre mécanostructurée par mécano-synthèse de poudre micrométrique de nacre selon la revendication 1, **caractérisé en ce que** les particules de nacre à traiter proviennent du test nacré de bivalve choisi dans le groupe comprenant :
la Pinctada Maxima, la Pinctada Margaritifera ou autres Pinctadines, le Tridacnae Gigas, et leurs mélanges.

3. Procédé de préparation de nacre mécanostructurée par mécano-synthèse de poudre micrométrique de nacre selon la revendication 1 ou 2 comprenant les étapes successives suivantes :
a) la poudre de nacre à traiter est placée dans un bol de broyage d'un broyeur planétaire, puis
b) Nᵢ billes de broyage (avec i est un nombre entier compris entre 1 et 20, de préférence entre 3 et 15 et plus préférentiellement entre 5 et 14 ; Nᵢ est un nombre entier compris entre 2 et 150, de préférence entre 10 et 100 et plus préférentiellement entre 20 et 85) de diamètre Dᵢ sont placées dans le bol de broyage,
c) le broyeur planétaire est mis en route, à une vitesse de rotation V comprise entre 800 et 1400 tr/min, préférentiellement à 1100 tr/min, avec une accélération de 90 à 110 G, de préférence de 90 à 100 G et plus préférentiellement de 95 G,
d) le broyeur planétaire est arrêté et les billes de broyage de diamètre Dᵢ sont retirées,
les étapes b, c et d sont répétées avec Nᵢ₊₁ (avec Nᵢ₊₁ > Nᵢ) billes de broyage de diamètre Dᵢ₊₁ (avec Dᵢ₊₁ < Dᵢ), jusqu'à l'obtention de particules de nacre de la taille souhaitée ;
lorsque la taille souhaitée des particules est obtenue, à l'issue de l'étape d), la nacre mécanostructurée est récupérée.

4. Procédé de préparation de nacre mécanostructurée par mécano-synthèse de poudre micrométrique de nacre selon l'une quelconque des revendications 1 à 3, **caractérisé par le fait que** le nombre de billes et leur diamètre sont tels que l'on ait un rapport 2/5 de poids de particules de nacre à traiter pour 3/5 de poids de billes.

5. Procédé de préparation de nacre mécanostructurée par mécano-synthèse de poudre micrométrique de nacre selon l'une quelconque des revendications 1 à 4, **caractérisé en ce que** le bol de broyage, la poudre de nacre à traiter et/ou les billes de broyage sont refroidis avant utilisation à une température comprise entre -30°C et 5°C, de préférence entre -20°C et -15°C et éventuellement avant chaque répétition des étapes b), c) et d).

6. Procédé de préparation de nacre mécanostructurée par mécano-synthèse de poudre micrométrique de nacre selon l'une quelconque des revendications 1 à 5, **caractérisé en ce que** les séquences de broyage c) sont effectuées sous atmosphère refroidie ou entrecoupées de séquences de refroidissement.

7. Procédé de préparation de nacre mécanostructurée par mécano-synthèse de poudre micrométrique de nacre selon l'une quelconque des revendications 1 à 6, **caractérisé en ce que** :
- la poudre de nacre à traiter est déposée dans un bol de broyage, des billes de broyage sont déposées dans le bol de broyage et l'ensemble comprenant la poudre de nacre à traiter, les billes de broyage et le bol de broyage est refroidi à une température comprise entre -30°C et 5°C, de préférence entre -20°C et -15°C,
- la poudre de nacre à traiter réfrigérée subit ensuite :
a) des séquences de broyage avec des billes de broyage de 10 mm de diamètre jusqu'à l'obtention de particules de nacre ayant un diamètre moyen en volume compris entre 5 et 15 microns, puis
b) des séquences de broyage avec des billes de broyage de 5 mm de diamètre, jusqu'à l'obtention de particules de nacre ayant un diamètre moyen en volume compris entre 800 nm et 2 µm, puis
c) des séquences de broyage avec des billes de broyage de 2 mm de diamètre, jusqu'à l'obtention de particules de nacre ayant un diamètre moyen en volume inférieur à 500 nanomètres, de préférence inférieur à 250 nm et plus préférentiellement inférieur à 100 nm,
la nacre nanostructurée est alors récupérée.

8. Procédé de préparation de nacre mécanostructurée par mécano-synthèse de poudre micrométrique de nacre selon l'une quelconque des revendications 1 à 7, **caractérisé en ce que** :
- la poudre de nacre à traiter est déposée dans un bol de broyage, des billes de broyage sont déposées dans le bol de broyage et l'ensemble comprenant la poudre de nacre à traiter, les billes de broyage et le bol de broyage est refroidi à une température comprise entre -30°C et 5°C, de préférence entre -20°C et -15°C,
- la poudre de nacre réfrigérée subit ensuite :
a) 5 à 15, de préférence 7 à 13 séquences, et plus préférentiellement encore 8 à 11 séquences de broyage, chaque séquence ayant une durée de 1 à 10 minutes, de préférence de 2 à 8 minutes et plus préférentiellement de 6 minutes, effectuées avec des billes de broyage de 10 mm de diamètre, puis
b) 5 à 15 séquences de broyage, préférentiellement 10 séquences, de 1 à 10 minutes, préférentiellement 6 minutes, effectuées avec des billes de broyage de 5 mm de diamètre, puis
c) 5 à 15 séquences de broyage, préférentiellement 10 séquences, de 1 à 10 minutes, préférentiellement 6 minutes, effectuées avec des billes de broyage de 2 mm de diamètre,
la nacre mécanostructurée est alors récupérée.

9. Poudre de nacre pouvant être obtenue par le procédé défini à l'une quelconque des revendications 1 à 8 et présentant un diamètre moyen en volume compris entre 0,01 et 500 nanomètres, de préférence entre 0,01 et 250 nm et plus préférentiellement entre 0,01 et 100 nm.

10. Procédé de préparation de nacre mécanostructurée par mécano-synthèse de poudre micrométrique de nacre selon l'une quelconque des revendications 1 à 8, **caractérisé en ce que** la poudre de nacre à traiter est broyée conjointement avec au moins un matériau autre que la nacre.

11. Procédé de préparation de nacre mécanostructurée par mécano-synthèse de poudre micrométrique de nacre selon la revendication 10, **caractérisé en ce que** le matériau est choisi dans le groupe comprenant la poudre de chitosan desacétylé à plus de 90%, la chitine, les algues, les biopolymères solubles et insolubles extraits du test nacré et de la coquille externe des bivalves précités, le sulfate de cuivre (CuSO₄, 5H₂O), l'oxyde de zinc, l'or ou l'argent, et leurs mélanges.

12. Utilisation de la nacre mécanostructurée selon la revendication 9 ou obtenue selon un procédé défini à l'une quelconque des revendications 1 à 8 et 10 à 11, dans les applications cosmétiques.

13. Utilisation de la nacre mécanostructurée selon la revendication 9 ou préparée selon le procédé défini à l'une quelconque des revendications 1 à 8 et 10 à 11 en revêtement d'implant osseux, dentaires, de pièces de substitution osseuse.

14. Implant comprenant un noyau en matériau de structure éventuellement en nacre, à la surface duquel est déposée en revêtement, par pulvérisation, projection, enrobage, électrolyse ou trempage, de la nacre selon la revendication 9 ou obtenue selon un procédé défini à l'une quelconque des revendications 1 à 8 et 10 à 11.

15. Pièce de comblement osseux comprenant un noyau en matériau de structure éventuellement en nacre, à la surface duquel est déposée en revêtement, par pulvérisation, projection, enrobage, électrolyse ou trempage, de la nacre selon la revendication 9 ou obtenue selon un procédé défini à l'une quelconque des revendications 1 à 8 et 10 à 11.

16. Nacre mécanostructurée selon la revendication 9 ou obtenue selon un procédé défini à l'une quelconque des revendications 1 à 8 et 10 à 11, pour une utilisation médicale ou vétérinaire.

## Patentansprüche

1. Verfahren zur Herstellung von mechanisch strukturiertem Perlmutt durch Mechanosynthese aus Perlmutt-Mikrometerpulver, **dadurch gekennzeichnet, dass** die Temperatur des Perlmutts unter 40 °C gehalten wird, vorzugsweise unter 20 °C und noch mehr bevorzugt unter oder bei gleich 0 °C gehalten wird, und dadurch, dass die zu behandelnden Perlmuttteilchen von Test-Perlmutt stammen, d.h. von der Innenschale von Muschel.

2. Verfahren zur Herstellung von mechanisch strukturiertem Perlmutt durch Mechanosynthese aus Perlmutt-Mikrometerpulver nach Anspruch 1, **dadurch gekennzeichnet, dass** die zu behandelnden Perlmuttteilchen von Muschel-Test-Perlmutt stammen, ausgewählt aus der Gruppe, umfassend:
Pinctada Maxima, Pinctada Margaritifera oder andere Pinctadine, Tridacnae Gigas und ihre Mischungen.

3. Verfahren zur Herstellung von mechanisch strukturiertem Perlmutt durch Mechanosynthese aus Perlmutt-Mikrometerpulver nach Anspruch 1 oder 2, umfassend die nachfolgenden aufeinanderfolgenden Schritte:
a) das zu behandelnde Perlmuttpulver wird in einer Mahlschale einer Planetenmühle angeordnet, dann
b) Nᵢ-Mahlkugeln (wobei i eine ganze Zahl ist, umfassend zwischen 1 und 20, vorzugsweise zwischen 3 und 15, mehr bevorzugt zwischen 5 und 14; Nᵢ eine Zahl ist, umfassend zwischen 2 und 150, vorzugsweise zwischen 10 und 100 und mehr bevorzugt zwischen 20 und 85) mit Durchmesser Dᵢ werden in dem Mahlbecher angeordnet;
c) die Planetenmühle wird mit einer Rotationsgeschwindigkeit V, umfassend zwischen 800 und 1400 U/min, vorzugsweise 1100 U/min betrieben, mit einer Beschleunigung von 90 bis 110 G, vorzugsweise von 90 bis 100 G und mehr bevorzugt 95 G,
d) die Planetenmühle wird angehalten und die Mahlkugeln mit Durchmesser Dᵢ werden entnommen,
die Schritte b, c und d werden wiederholt mit Nᵢ₊₁ (wobei Nᵢ₊₁ > Nᵢ) Mahlkugeln mit Durchmesser Dᵢ₊₁ (wobei Dᵢ₊₁ < Dᵢ), bis zum Erhalt von Perlmuttteilchen mit der gewünschten Größe;
wobei, wenn die gewünschte Größe der Teilchen erreicht ist, nach Abschluss von Schritt d), das mechanisch strukturierte Perlmutt zurückgewonnen wird.

4. Verfahren zur Herstellung von mechanisch strukturiertem Perlmutt durch Mechanosynthese von Perlmutt-Mikrometerpulver nach einem der Ansprüche 1 bis 3, **dadurch gekennzeichnet, dass** die Anzahl der Kugeln und ihr Durchmesser so ist, dass man ein Verhältnis 2/5 des Gewichts der zu behandelnden Perlmuttteilchen auf 3/5 des Gewichts der Kugeln hat.

5. Verfahren zur Herstellung von mechanisch strukturiertem Perlmutt durch Mechanosynthese von Perlmutt-Mikrometerpulver nach einem der Ansprüche 1 bis 4, **dadurch gekennzeichnet, dass** der Mahlbecher, das zu behandelnde Perlmuttpulver und/oder die Mahlkugeln vor der Verwendung abgekühlt werden auf eine Temperatur, umfassend zwischen -30 °C und 5 °C, vorzugsweise zwischen -20 °C und -15°C, und gegebenenfalls vor jeder Wiederholung der Schritte b), c) und d).

6. Verfahren zur Herstellung von mechanisch strukturiertem Perlmutt durch Mechanosynthese aus Perlmutt-Mikrometerpulver nach einem der Ansprüche 1 bis 5, **dadurch gekennzeichnet, dass** die Mahlsequenzen c) unter einer kühlen Atmosphäre oder unterbrochen von Abkühlsequenzen durchgeführt werden.

7. Verfahren zur Herstellung von mechanisch strukturiertem Perlmutt durch Mechanosynthese aus Perlmutt-Mikrometerpulver nach einem der Ansprüche 1 bis 6, **dadurch gekennzeichnet, dass**:
- das zu behandelnde Perlmuttpulver in einen Mahlbecher eingebracht wird, Mahlkugeln in den Mahlbecher eingebracht werden und das Gesamte, umfassend das zu behandelnde Perlmuttpulver, die Mahlkugeln und den Mahlbecher, abgekühlt wird auf eine Temperatur, umfassend zwischen -30 °C und 5 °C, vorzugsweise zwischen -20 °C und -15 °C,
- das abgekühlte zu behandelnde Perlmuttpulver dann Folgendes durchläuft:
a) Mahlsequenzen mit Mahlkugeln mit 10 mm Durchmesser bis zum Erhalten von Perlmuttteilchen mit einem mittleren volumenbezogenen Durchmesser, umfassend zwischen 5 und 15 Mikrometer, dann
b) Mahlsequenzen mit Mahlkugeln mit 5 mm Durchmesser bis zum Erhalten von Perlmuttteilchen mit einem mittleren volumenbezogenen Durchmesser, umfassend zwischen 800 nm und 2 µm, dann
c) Mahlsequenzen mit Mahlkugeln mit 2 mm Durchmesser bis zum Erhalten von Perlmuttteilchen mit einem mittleren volumenbezogenen Durchmesser von 500 Nanometer, vorzugsweise unter 250 nm und mehr bevorzugt unter 100 nm,
wobei das nanostrukturierte Perlmutt dann zurückgewonnen wird.

8. Verfahren zur Herstellung von mechanisch strukturiertem Perlmutt durch Mechanosynthese aus Perlmutt-Mikrometerpulver nach einem der Ansprüche 1 bis 7, **dadurch gekennzeichnet, dass**:
- das zu behandelnde Perlmuttpulver in einen Mahlbecher eingebracht wird, Mahlkugeln in den Mahlbecher eingebracht werden und das Gesamte, umfassend das zu behandelnde Perlmuttpulver, die Mahlkugeln und den Mahlbecher, abgekühlt wird auf eine Temperatur, umfassend zwischen -30 °C und 5 °C, vorzugsweise zwischen -20 °C und -15 °C,
- das abgekühlte zu behandelnde Perlmuttpulver dann Folgendes durchläuft:
a) 5 bis 15, vorzugsweise 7 bis 13 Sequenzen und noch mehr bevorzugt 8 bis 11 Mahlsequenzen, wobei jede Sequenz 1 bis 10 Minuten, vorzugsweise 2 bis 8 Minuten und mehr bevorzugt 6 Minuten dauert, durchgeführt mit Mahlkugeln mit 10 mm Durchmesser, dann
b) 5 bis 15 Mahlsequenzen, vorzugsweise 10 Sequenzen von 1 bis 10 Minuten, vorzugsweise 6 Minuten, durchgeführt mit Mahlkugeln mit 5 mm Durchmesser, dann
c) 5 bis 15 Mahlsequenzen, vorzugsweise 10 Sequenzen von 1 bis 10 Minuten, vorzugsweise 6 Minuten, durchgeführt mit Mahlkugeln mit 2 mm Durchmesser,
wobei das mechanisch strukturierte Perlmutt dann zurückgewonnen wird.

9. Perlmuttpulver, das durch das Verfahren nach einem der Ansprüche 1 bis 8 erhalten werden kann und einen mittleren volumenbezogenen Durchmesser aufweist, umfassend zwischen 0,01 und 500 Nanometer, vorzugsweise zwischen 0,01 und 250 nm und mehr bevorzugt zwischen 0,01 und 100 nm.

10. Verfahren zur Herstellung von mechanisch strukturiertem Perlmutt durch Mechanosynthese aus Perlmutt-Mikrometerpulver nach einem der Ansprüche 1 bis 8, **dadurch gekennzeichnet, dass** das zu behandelnde Perlmuttpulver zusammen mit mindestens einem von Perlmutt verschiedenem Material gemahlen wird.

11. Verfahren zu Herstellung von mechanisch strukturiertem Perlmutt durch Mechanosynthese aus Perlmutt-Mikrometerpulver nach Anspruch 10, **dadurch gekennzeichnet, dass** das Material ausgewählt wird aus der Gruppe, umfassend 90 % deacetyliertes Chitosan, Chitin, Algen, lösliche Biopolymere und unlösliche Extrakte von Test-Perlmutt und Außenschale oben genannter Muscheln, Kupfersulfat (CuSO₄, 5 H₂O), Zinkoxid, Gold oder Silber und ihre Mischungen.

12. Verwendung des mechanisch strukturierten Perlmutts nach Anspruch 9 oder des Perlmutts welcher erhalten wurde nach einem Verfahren nach einem der Ansprüche 1 bis 8 und 10 bis 11 in kosmetischen Anwendungen.

13. Verwendung des mechanisch strukturierten Perlmutts nach Anspruch 9 oder des Perlmutts, welcher erhalten wurde nach einem Verfahren nach einem der Ansprüche 1 bis 8 und 10 bis 11 in einer Beschichtung auf Knochenimplantat, Zähnen, Knochenersatzmaterialteilen.

14. Implantat, umfassend einen Kern aus Strukturmaterial, gegebenenfalls aus Perlmutt, auf dessen Oberfläche eine Beschichtung aufgebracht ist durch Pulverisieren, Aufspritzen, Ummantelung, Elektrolyse oder Einweichen, aus dem Perlmutt nach Anspruch 9 oder erhalten nach einem Verfahren nach einem der Ansprüche 1 bis 8 und 10 bis 11.

15. Knochenfüllungsteil, umfassend einen Kern aus einem Strukturmaterial, gegebenenfalls aus Perlmutt, auf dessen Oberfläche durch Beschichten, durch Pulverisieren, Aufspritzen, Ummantelung, Elektrolyse oder Einweichen, Perlmutt nach Anspruch 9 oder nach einem Verfahren nach einem der Ansprüche 1 bis 8 und 10 bis 11 aufgebracht ist.

16. Mechanisch strukturiertes Perlmutt nach Anspruch 9 oder erhalten nach einem Verfahren nach einem der Ansprüche 1 bis 8 und 10 bis 11, für eine medizinische oder veterinäre Anwendung.

## Claims

1. Process for the preparation of mechano-structured nacre by mechanosynthesis of micrometric nacre powder, **characterized in that** the temperature of the nacre is kept below 40°C, preferably below 20°C, and even more preferentially below or equal to 0°C, and **in that** the nacre particles to be treated are obtained from the nacreous test, i.e. the inner shell of bivalves.

2. Process for the preparation of mechano-structured nacre by mechanosynthesis of micrometric nacre powder according to claim 1, **characterized in that** the nacre particles to be treated are obtained from the nacreous test of bivalves chosen from the group comprising:
Pinctada maxima, Pinctada margaritifera or other Pinctadas, Tridacnae gigas, and mixtures thereof.

3. Process for the preparation of mechano-structured nacre by mechanosynthesis of micrometric nacre powder according to claim 1 or 2 comprising the following successive stages:
a) the nacre powder to be treated is placed in a grinding bowl of a planetary mill, then
b) Nᵢ grinding beads (with i an integer comprised between 1 and 20, preferably between 3 and 15 and more preferentially between 5 and 14; Nᵢ is an integer comprised between 2 and 150, preferably between 10 and 100 and more preferentially between 20 and 85) of diameter Dᵢ are placed in the grinding bowl,
c) the planetary mill is started up at a rotational speed V comprised between 800 and 1400 rpm, preferentially at 1100 rpm, with an acceleration of 90 to 110 G, preferably of 90 to 100 G and more preferentially of 95 G,
d) the planetary mill is stopped and the grinding beads of diameter Dᵢ are removed,
Stages b, c and d are repeated with Nᵢ₊₁ (with Nᵢ₊₁ > Nᵢ) grinding beads of diameter Dᵢ₊₁ (with Dᵢ₊₁ < Dᵢ), until nacre particles of the desired size are obtained;
when the desired size of the particles is obtained, on completion of stage d), the mechano-structured nacre is recovered.

4. Process for the preparation of mechano-structured nacre by mechanosynthesis of micrometric nacre powder according to any one of claims 1 to 3, **characterized by** the fact that the number of beads and their diameter are such that there is a ratio of 2/5 by weight of nacre particles to be treated to 3/5 by weight of beads.

5. Process for the preparation of mechano-structured nacre by mechanosynthesis of micrometric nacre powder according to any one of claims 1 to 4, **characterized in that** the grinding bowl, the nacre powder to be treated and/or the grinding beads are cooled down before use to a temperature comprised between -30°C and 5°C, preferably between -20°C and -15°C and optionally before each repetition of stages b), c) and d).

6. Process for the preparation of mechano-structured nacre by mechanosynthesis of micrometric nacre powder according to any one of claims 1 to 5, **characterized in that** the grinding cycles c) are carried out under a cooled atmosphere or interspersed with cooling cycles.

7. Process for the preparation of mechano-structured nacre by mechanosynthesis of micrometric nacre powder according to any one of claims 1 to 6, **characterized in that**:
- the nacre powder to be treated is placed in a grinding bowl, grinding beads are placed in the grinding bowl and the assembly comprising the nacre powder to be treated, the grinding beads and the grinding bowl is cooled down to a temperature comprised between -30°C and 5°C, preferably between -20°C and -15°C,
- the refrigerated nacre powder to be treated then undergoes:
a) grinding cycles with grinding beads 10 mm in diameter until nacre particles having an mean volume diameter comprised between 5 and 15 microns are obtained, then
b) grinding cycles with grinding beads 5 mm in diameter, until nacre particles having an mean volume diameter comprised between 800 nm and 2 µm are obtained, then
c) grinding cycles with grinding beads 2 mm in diameter, until nacre particles having an mean volume diameter of less than 500 nanometres, preferably less than 250 nm and more preferentially less than 100 nm are obtained,
the nanostructured nacre is then recovered.

8. Process for the preparation of mechano-structured nacre by mechanosynthesis of micrometric nacre powder according to any one of claims 1 to 7, **characterized in that**:
- the nacre powder to be treated is placed in a grinding bowl, grinding beads are placed in the grinding bowl and the assembly comprising the nacre powder to be treated, the grinding beads and the grinding bowl is cooled down to a temperature comprised between -30°C and 5°C, preferably between -20°C and -15°C,
- the refrigerated nacre powder then undergoes:
a) 5 to 15, preferably 7 to 13 cycles, and even more preferentially 8 to 11 grinding cycles, each cycle lasting 1 to 10 minutes, preferably of 2 to 8 minutes and more preferentially of 6 minutes, carried out with grinding beads 10 mm in diameter, then
b) 5 to 15 grinding cycles, preferentially 10 cycles, of 1 to 10 minutes, preferentially 6 minutes, carried out with grinding beads 5 mm in diameter, then
c) 5 to 15 grinding cycles, preferentially 10 cycles, of 1 to 10 minutes, preferentially 6 minutes, carried out with grinding beads 2 mm in diameter,
the mechano-structured nacre is then recovered.

9. Nacre powder which can be obtained by the process defined in any one of claims 1 to 8 and having an mean volume diameter comprised between 0.01 and 500 nanometres, preferably between 0.01 and 250 nm and more preferentially between 0.01 and 100 nm.

10. Process for the preparation of mechano-structured nacre by mechanosynthesis of micrometric nacre powder according to any one of claims 1 to 8, **characterized in that** the nacre powder to be treated is ground together with at least one material other than the nacre.

11. Process for the preparation of mechano-structured nacre by mechanosynthesis of micrometric nacre powder according to claim 10, **characterized in that** the material is chosen from the group comprising more than 90%-deacetylated chitosan powder, chitin, algae, insoluble and soluble biopolymers extracted from the nacreous test and from the outer shell of the abovementioned bivalves, copper sulphate (CuSO₄, 5H₂O), zinc oxide, gold or silver, and mixtures thereof.

12. Use of the mechano-structured nacre according to claim 9 or obtained according to a process defined in any one of claims 1 to 8 and 10 to 11, in cosmetic applications.

13. Use of the mechano-structured nacre according to claim 9 or prepared according to the process defined in any one of claims 1 to 8 and 10 to 11 in coating of bone and dental implants, and bone substitute pieces.

14. Implant comprising a core of structural material, optionally of nacre, the surface of which is covered by sputtering, spraying, coating, electrolysis or immersion of the nacre according to claim 9 or obtained according to a process defined in any one of claims 1 to 8 and 10 to 11.

15. Bone filling piece comprising a core of structural material optionally made of nacre, the surface of which is covered by sputtering, spraying, coating, electrolysis or immersion of the nacre according to claim 9 or obtained according to a process defined in any one of claims 1 to 8 and 10 to 11.

16. Mechano-structured nacre according to claim 9 or obtained according to a process defined in any one of claims 1 to 8 and 10 to 11, for a medical or veterinary use.
